Europäisches Patentamt

European Patent Office

Office européen des brevets

(11) Veröffentlichungsnummer: **0 430 193 A1**

## (12) EUROPÄISCHE PATENTANMELDUNG

(21) Anmeldenummer: 90122694.4

(22) Anmeldetag: 28.11.90

(51) Int. Cl.5: **C12N 5/20**, C12P 21/08, A61K 39/395, G01N 33/577, G01N 33/68

(30) Priorität: 01.12.89 DE 3939706

(43) Veröffentlichungstag der Anmeldung:
05.06.91 Patentblatt 91/23

(84) Benannte Vertragsstaaten:
AT BE CH DE DK ES FR GB GR IT LI LU NL SE
Patentblatt

(71) Anmelder: CENTRE REGIONAL DE
TRANSFUSION SANGUINE
1 Bld Alexandre Fleming BP 1937
F-25020 Besancon(FR)

Anmelder: BIOTEST PHARMA GMBH
Landsteiner Strasse 5
W-6072 Dreieich(DE)

(72) Erfinder: Wijdenes, John, Prof.
Rue du Coteau Auxon-Dessus
F-25870 Geneuille(FR)
Erfinder: Clement, Claude

Essertenne
F-70100 Gray(FR)
Erfinder: Morel-Fourrier, Brigitte
2 Rue Leonard de Vinci
F-25000 Besancon(FR)
Erfinder: Peters, Andre, Prof.
2 Place Jean Cornet
F-25000 Besancon(FR)
Erfinder: Kloft, Michael, Dr .
Greinstrasse 18
W-6100 Darmstadt(DE)
Erfinder: Sebald, Walter, Prof.Dr.
Meyer-Olbersleben-Strasse 5
W-8700 Würzburg(DE)
Erfinder: Schwulera, Udo, Dr.
Krebsbachweg 23
W-6450 Hanau 1(DE)

(74) Vertreter: Dr. Fuchs, Dr. Luderschmidt
Dipl.-Phys. Seids, Dr. Mehler Patentanwälte
Abraham-Lincoln-Strasse 7, Postfach 4660
W-6200 Wiesbaden(DE)

(54) **Monoklonale Antikörper und ihre Verwendung.**

(57) Die Erfindung betrifft 3 Hybridoma-Zellinien, nämlich BE-8 (C.N.C.M.-Nr.I/913), BE-4 (C.N.C.M.-Nr.I/911) und BF-6 (C.N.C.M.-Nr.I/912), die durch Fusion von immunisierten Milzzellen der Maus mit Maus-Myelomzellen erhältlich sind, nach dem die Milzzellen vor der Fusion gegen Interleukin-6 immunisiert worden waren. Diese 3 Zellinien produzieren monoklonale Antikörper, welche humanes Interleukin-6 erkennen und in Abhängigkeit von den unterschiedlichen Zellinien jeweils an ein anderes Epitop auf dem Interleukin-6 Molekül bilden.

Die monoklonalen Antikörper lassen sich an Toxine und/oder Chemotherapeutika koppeln und können bei der Herstellung einer Komposition zur Therapie, Prophylaxe and Diagnose von Interleukin-6-abhängigen Erkrankungen verwendet werden.

EP 0 430 193 A1

# MONOKLONALE ANTIKÖRPER UND IHRE VERWENDUNG

Die Erfindung betrifft die in den Ansprüchen 1 und 2 angegebenen Hybridoma-Zellinien und davon produzierte monoklonale Antikörper nach den Ansprüchen 3-6 sowie die Verwendung dieser monoklonalen Antikörper nach Anspruch 7.

Interleukin-6 (IL-6), ursprünglich auch als humaner B-Zell-Stimulationsfaktor 2 (BSF-2) (M. Kawano, T. Hirano et al., Nature, Vol. 332, 3, 83-85, 1988) oder Hybridoma Growth Factor (HGF) (R. Bazin und R. Lemieux, J. Immunol. 139, 780-787, 1987) bezeichnet, gehört zu den Mediatoren des zellulären Immunsystems.

Die Sequenz der IL-6-codierenden cDNA und die daraus resultierende Aminosäuresequenz (184 Aminosäuren) ist in der Literatur beschrieben (T. Hirano et al., Nature 324, 6, 73-76, 1987).

Es ist bekannt, daß Interleukin-6 (IL-6) ein weites Spektrum biologischer Funktionen besitzt. Wirkungen von Interleukin-6 auf T-Zellen (R. D. Gorman et al., Proc. natn. Acad. Sci. USA 84, 7629-7633, 1987), Plasmacytome (J. van Damme et al., J. exp. Med. 165, 914-919, 1987); Hepatozyten (T. Andus et al., FEBs Lett. 221, 18-22, 1987) und Fibroblasten (M. Kohase et al., Cell 45, 659-666, 1986) sind in der Literatur eindeutig beschrieben.

Die Rolle von IL-6 und von IL-6-Antagonisten wird in den folgenden Übersichtsarbeiten ausführlich beschrieben:

"Regulation of the acute phase and immune responses"

"Interleukin-6",

Editor P.B. Sehgal et al.,

The New York Academy of Sciences, 2, East 63rd Street, New York, USA

und

T. Andus et al., DMW 44, 1989

Die Bedeutung von IL-6 bei der Entstehung der verschiedensten Krankheitsbilder und ihrer Verläufe und die Beeinflussung durch IL-6-Antagonisten (IL-6-Antikörper) bei hämatologischen und soliden Tumoren, bei Autoimmunerkrankungen und entzündlichen Prozessen, bei viralen, bakteriellen und mykotischen Infektionen, bei Störungen der Acute-Phase-Reaktionen und bei der Beeinflussung der Lymphokin-Kaskade sind in obiger Literatur zusammengefaßt.

Daneben gibt es eine Vielzahl von Literaturbeispielen zu der pathophysiologischen Rolle von IL-6, von denen hier nur eine begrenzte Anzahl beispielhaft zitiert wird:

So beschreiben C. P. Chin und F. Lee (J. of Immun., 142, 1909-1915, 1989) die Rolle von IL-6 bei der Regulation von Wachstum und Differenzieren myeloischer Leukämiezellen.

P.A. Guerne et al. (J. Clin. Invest., 83, 585-592, 1989) zeigen, daß besonders hohe Spiegel von IL-6 bei Patienten mit entzündlichen Arthropathien gefunden werden und offensichtlich eine bedeutende Rolle im Verlauf dieser Krankheitsbilder spielen.

C. E. Hack (Blood, 74, No. 15, 1704-1710, 1989) konnten erstmals zeigen, daß Interleukin-6 eine bedeutende Rolle in der Pathophysiologie des septischen Schocks spielt. Sie fanden bei Untersuchungen, daß die Mehrzahl der Patienten mit septischem Schock stark erhöhete IL-6-Spiegel im Vergleich zum Normalkollektiv aufwiesen.

A. J. G. Swaak et al. (Rheumatol. Int. 8, 263-268, 1989) fanden bei Untersuchungen über den IL-6-Spiegel bei Patienten mit Systemischem Lupus erythematodes Korrelationen zwischen IL-6-Menge und akute-Phase-Protein-Antwort. Diese Untersuchungen und andere Untersuchungen zeigen, daß IL-6 wahrscheinlich verantwortlich ist für die Induktion der Produktion von Akute-Phase-Proteinen durch Hepatozyten.

Kastleman's Syndrom ist eine Erkrankung, die durch Lymphocytenhyperblasie, Plasmazellinfiltration, Fieber, Anämie und Hypergammaglobulinaemie gekennzeichnet ist. T. Nishimoto et al. (Blood 4, 1360-1367, 1989) konnten zeigen, daß bei dieser Erkrankung eine klare Korrelation zwischen dem Serumspiegel von IL-6 und dem klinischen Bild der Erkrankung besteht und das Il-6 als das Schlüsselelement für die Auslösung der verschiedenen klinischen Symptome angesehen werden muß.

Dieselbe Arbeitsgruppe um T. Kishimoto (FEBs Lett. 250, 607-610, 1989) konnte zeigen, daß IL-6 ein autokriner Wachstumsfaktor für Nierenzellkarzinome ist und daß das Wachstum dieser Tumorzellen durch ein Anti-IL-6-Antiserum inhibiert werden kann. Die Rolle von IL-6 als der entscheidende Wachstumsfaktor für Myelomzellen wurde von einer ganzen Reihe von Arbeitsgruppen untersucht:

X. G. Zhang et al. (Blood, 74, 11-13, 1989) konnten zeigen, daß der IL-6 response von Myelomzellen in vitro direkt mit dem Proliferationsstatus in vivo und damit mit der Schwere der Erkrankung korreliert.

L. Bergin et al. (J. Exp. Med., 170, 613-618, 1989) konnten zeigen, daß IL-6 nicht nur auf die maligne ausgereifte Zelle wirkt, sondern daß IL-6 die Proliferation und das Differenzieren von malignen Plasmazellvorstufen in Multiplen Myelomen unterstützt.

T. Kishimoto et al. (Nature, 332, 83-85, 1988) konnten zum ersten Mal experimentell nachweisen, daß es möglich ist, das Wachstum von IL-6-abhängigen Myelomzellen mit Anti-IL-6-Antikörpern zu hemmen.

In der PCT-Schrift WO 88/00 206 ist die Herstellung und die Verwendung von IL-6 beschrieben, wobei auch darauf hingewiesen wird, daß unter Verwendung von IL-6 in an sich bekannten Verfahren polyklonale und monoklonale Antikörper hergestellt werden können. Ein Weg zur Herstellung derartiger Antikörper ist jedoch nicht beschrieben. Ebenfalls ist kein Antikörper durch chemisch/physikalische Parameter angegeben, noch eine Zelle hinterlegt worden, mit der ein solcher Antikörper produziert werden kann. Insofern fehlt es an einer nachvollziehbaren Lehre, d.h. die Offenbarung dieser PCT-Schrift erschöpft sich in rein spekulativen Erwägungen.

Das Ziel dieser Erfindung ist es, monoklonale Antikörper herzustellen, die in der Lage sind, effektiv das Wachstum IL-6-abhängiger Zellen zu inhibieren oder zu unterdrücken. Diese monoklonalen Antikörper können zur Herstellung einer Zusammensetzung verwendet werden, die sowohl therapeutisch als auch prophylaktisch in niedrigen Dosen eingesetzt werden kann, so daß keine Nebenwirkungen auftreten.

Dieses Ziel wurde erreicht durch die Anwendung dem bekannten Fusionsmethode - entwickelt von C. Milstein und G. Köhler - und Isolierung neuer Hybridomazellinien, die Maus-monoklonale Antikörper gegen IL-6 produzieren.

Aus diesen Zellinien mit der Bezeichnung BE-4 (IgG2b); BE-8 (IgGI) und BF-6 (IgGI), die bei der Nationalen Französichen Sammlung for Mikroorganismen (CNCM) unter den Nummern I/911, I/913, I/912 hinterlegt sind, können Class-Switch-Varianten des Maus-Immunglobulins, wie z. B. IgG2a, IgG2b, IgG3, IgG1 und andere Immunglobulinklassen, isoliert werden.

Die monoklonalen Antikörper BE-4 und BE-8 konkurrieren mit IL-6 um die Bindung an den IL-6-Rezeptor auf humanen und Maus-Zellinien und inhibieren die Proliferation von IL-6-abhängigen Zellinien.

Beide Antikörper sind in der Lage, IL-6, welches an den Rezeptor gebunden ist, zu erkennen, und es wurde gezeigt, daß BE-4 und BE-8 unterschiedlich Epitope auf den IL-6-Molekülen erkennen.

Der monoklonale Antikörper BF-6 erkennt ebenfalls IL-6, aber konkurriert nicht mit IL-6 um die Bindung an den IL-6-Rezeptor, noch ist BF-6 in der Lage, die Proliferation von IL-6-abhängigen Zellen zu hemmen. BF-6 ist fähig, an den Rezeptor gebundenes IL-6 zu erkennen, und es wurde gezeigt, daß er ein Epitop erkennt, welches verschieden ist von den durch BE-4 und BE-8 erkannten Epitopen.

Die monoklonalen Antikörper sind deshalb geeignet bei der Herstellung einer Zusammensetzung für die Behandlung solcher Erkrankungen wie Multiples Myelom, Myeloische Leukämie, Kastleman's Symdrom, Systemischer Lupus Erythematodes, Nierenzellkarzinome, entzündliche Arthropathie und andere Erkrankungen verwendet zu werden, von denen gezeigt wurde, daß sie IL-6-abhängig sind.

Die monoklonalen Antikörper können als reine Substanz, gekoppelt mit Toxinen (wie Ricin A oder Saporin) oder radioaktiven Substanzen oder anderen Pharmazeutika, oder eingekapselt in Liposomen, benutzt werden.

Arzneimittel, die die Antikörper BE-4, BE-8 oder BF-6 enthalten, können in flüssiger oder lyophilisierter Form vorliegen. Zur Stabilisierung können verwendet werden Proteine, Zucker, Zuckeralkohole, Aminosäuren, viskositätssteigernde Agenzien sowie zur Pufferung anorganische Salze, vorzugsweise Na-Phosphat in physiologischem Milieu (PBS pH 7.4).

Die Antikörper werden in einer Konzentration von 0,5 - 5 mg/ml, vorzugsweise 1 mg/ml zur Herstellung einer Zusammensetzung für therapeutische Zwecke eingesetzt, die in der Regel systemisch appliziert wird, wobei jedoch lokale Applikationen nicht ausgeschlossen sind.

Die monoklonalen Antikörper BE-4, BE-8 und BF-6 können ebenso zur Herstellung von Zusammensetzungen verwendet werden, die als diagnostische Reagenzien für die Erkennung von IL-6 an seinen Rezeptor, auf der Oberfläche von Zellen, oder in Körperflüssigkeiten benutzt werden. Bei solcher Verwendung können die Antikörper mit fluoreszierenden oder anderen Substanzen gekoppelt werden. Es ist ebenso möglich, die Antikörper für einen ELISA oder Radioimmunassay zu verwenden, mit dem IL-6 in Körperflüssigkeiten gemessen wird.

Die monoklonalen Antikörper in Übereinstimmung mit der Erfindung sind dazu geeignet, daraus chimäre Antikörper mit der konstanten Domäne menschlichen Ursprungs (humanes Immunglobulin) und der variablen und insbesondere nur der hypervariablen Region aus murinem Ursprung (Maus-Immunglobin) zu produzieren. Die Chimären können als reine Substanz oder aber gekoppelt an Toxine, radioaktive Substanzen, andere Pharmazeutika oder eingekapselt in Liposome, zur Herstellung von Zusammensetzungen verwendet werden, die zur Behandlung von IL-6-abhängigen Erkrankungen dienen.

Die Erfindung wird im einzelnen durch die folgenden Beispiele beschrieben:


**I. Herstellung der monoklonalen Antikörper**

Beispiel 1: Immunisierung, Fusion, Klonierung und Gewinnung der monoklonalen Antikörper BE-4, BE-8 und BF-6

Weibliche Balb/c-Mäuse wurden intraperitoneal 4 mal in zweiwöchigem Abstand mit je 10 µg rekombinantem IL-6 immunisiert. Die vierte Immunisierung wurde intravenös verabreicht und die Milzzellen 4 Tage später entnommen und fusioniert. Die Fusion wurde wie folgt durchgeführt;

Die immunisierten Milzzellen wurden mit X63Ag8653 Maus-Myelomzellen im Verhältnis 5:1 in Gegenwart von Polyethylenglykol fusioniert (Kearney et al., J. of Immunol. 123, 1548, 1978). Diese Zellinie ist bei European Collection of Animal Cell Cultures (ECACC), PHLS Centre for Applied Microbiology and Research, Porton Down, Salisbury, Wiltshire, SP4 0IG, UK unter ECAAC No. 850 114 20 hinterlegt.

Die fusionierte Zellsuspension wurde einmal gewaschen und im Selektionsmedium (RPMI 1640, 10 % hitzeinaktiviertes Pferdeserum, 4 mM Glutamin, Hypoxanthin 13,6 mg/l, Aminopterin 0,17 mg/l und 10 µg/ml Insulin) kultiviert.

Zehn Tage nach der Fusion wurden Überstände von Kulturen, die Hybridomawachstum zeigten, auf Anti-IL-6-Mak-Produktion getestet.

Dazu wurden 100 µl von Hybridoma-Überständen für eine Stunde in einer ELISA-Platte inkubiert, die zuvor mit 1000 ng Anti-Maus-Immunglobulin bei 4° C über Nacht beschichtet wurde.

Nach dreimaligem Waschen wurden die einzelnen Löcher mit je 100 ng biotihyliertem IL-6 in 100 ml PBS für 1 Stunde bei Raumtemperatur inkubiert. Nach folgendem dreimaligen Waschen folgte eine einstündige Reaktion für 1 Stunde mit Streptavidin Peroxidase bei Raumtemperatur, nochmaliges Waschen und Inkubation mit Substrat (DPO). Anschließend wurden die Platten bei einer optischen Dichte von 405 nm vermessen. Die positiven Klone wurden nach 4 Klonierungsschritten unter Benutzung der "limiting dilution"-Methode (Aussaatdichte 0,2 Zellen/Kultur) untersucht und die Klone BE-4, BE-8 und BF-6 isoliert. BE-4 ist ein Maus-IgG2b-Antikörper, BE-6 und BE-8 sind IgG1-Antikörper und alle haben eine Kappa leichte Kette und zeigen eine signifikante Bindung an rekombinantes IL-6.

Beispiel 2: In-vivo-Produktion und Reinigung der Antikörper BE-4, BE-8 und BF-6

Die anti-IL-6-monoklonalen Antikörper BE-4, BE-8 und BF-6 wurden in großen Mengen in vivo durch intraperitoneale Injektion von je BE-4, BE-8 und BF-6-Hybridomazellen In Balb/c-Mäusen produziert. Eine Woche vor der Hybridomazellinjektion wurden die Mäuse mit 0,5 ml Freund'schem inkompletten Adjuvans intraperitoneal geprimt. 8 - 14 Tage nach der Hybridomazellinjektion konnte Ascitesflüssigkeit abgenommen werden.

Die Mak's wurden anschließend durch Ammoniumsulfatfällung (45 % Sättigung) aus der Ascitesflüssigkeit gefällt, auf 0,02 mM Tris, pH 7,7 umgepuffert und an eine Q-Sepharose-Säule gebunden. Auf diesen Säulen wurden die Mak's mit 1 % Tween 20 in 0,02 mM Tris, pH 7,7 gewaschen und anschließend mit 0,35 M NaCl-Lösung pH 7,7 von der Säule eluiert.

Für die Verwendung zur Herstelung einer Zusammensetzung für therapeutische Zwecke wurde der Mak auf physiologischen PBS-Puffer (phosphatgepufferte Saline) umgepuffert.

Beispiel 3: Inhibition der IL-6-induzierten Proliferation der IL-6-abhängigen Zellinie B9 durch BE-4, BE-8 und BF-6

Die Zellinie B9 ist eine Maushybridoma-Linie, die IL-6-abhängig ist und deren Eigenschaften durch L. A. van Aarden beschrieben wurden (Eur. J. Immunol. 17, 1411, 1987). Die Zellinie B9 wird in RPMI1608 mit 10 % foetalem Kälberserum und Mercaptoethanol unter Zugabe von 2 pg IL-6/ml für 3 Tage kultiviert. Anschließend wird für 16 Stunden $H^3$-Thymidin hinzugefügt. Anschließend werden die Zellen gesammelt, gewaschen und im Beta-Counter vermessen.

In den nachfolgenden Experimenten werden bei der Zugabe von 2 pg/ml IL-6 verschiedene Konzentrationen der Antikörper BE-4, BE-8 und BF-6 mit hinzugefügt. Die DNA-Synthese als ein Maß für das Zellwachstum oder Zellinhibition wurde als $H^3$-Thymidin-Einbau durch die Vermessung der Radioaktivität gemessen.

Gemessene Radioaktivität (cpm)

| | | IL-6 (0 pg/ml) | IL-6 (2 pg/ml) |
|---|---|---|---|
| BE-4 | 0 | 6910 | 115976 |
| | 1 pg | 7552 | 91601 |
| | 10 pg | 7881 | 28247 |
| | 100 pg | 7941 | 15384 |
| | 1 ng | 9055 | 8326 |
| | 10 ng | 8178 | 4860 |
| | 100 ng | 7611 | 5327 |
| | 1 µg | 6992 | 4315 |
| | | | |
| BE-8 | 0 | 8916 | 136217 |
| | 1 pg | 9002 | 64500 |
| | 10 pg | 8225 | 28002 |
| | 100 pg | 8264 | 16259 |
| | 1 ng | 9027 | 8281 |
| | 10 ng | 8356 | 5629 |
| | 100 ng | 9221 | 5434 |
| | 1 µg | 10836 | 7470 |

| | IL-6 (0 pg/ml) | | | IL-6 (2 pg/ml) |
|---|---|---|---|---|
| BF-6 | 0 | 7488 | | 126167 |
| | 1 pg | 8050 | | 131178 |
| | 10 pg | 8366 | | 116380 |
| | 100 pg | 9118 | | 118153 |
| | 1 ng | 7673 | | 124812 |
| | 10 ng | 7987 | | 119631 |
| | 100 ng | 8855 | | 115186 |
| | 1 ng | 9412 | | 117318 |

## cpm = disintegration per minute, Mittelwert von zwei Messungen

Die Ergebnisse zeigen eindeutig, daß der $H^3$-Thymidin-Einbau, und damit die DNA-Synthese als Maß des Zellwachstums, deutlich niedriger ist bei den B9-Kulturen, die mit BE-4 oder BE-8 geprüft wurden. Diese Antikörper inhibieren das IL-6-abhängige Wachstum der Zellinie B9. BF-6 dagegen ist nicht in der Lage, die Proliferation der Zellinie B-9 zu hemmen.

Beispiel 4: Scatchard-Analyse: Ermittlung der Affinitätskonstanten von IL-6 an die Zellinie U226
**Jodierung von IL-6:**

5 $\mu$g IL-6 in 10 $\mu$l Boratpuffer (0,1 M, pH 8,0) wurden mit 1 mCi $I^{125}$ für 15 Minuten bei 0° C inkubiert. Die Reaktion wurde dann mit 500 $\mu$l Glycin (0,2 M in Boratpuffer 0,1 M pH 8,5) für 5 Minuten abgestoppt.

Freies $I^{125}$ und gebundenes $I^{125}$ wurden über eine PD10-Säule, die vorher mit PBS 1 % Rinderalbumin equilibriert wurde, getrennt. Die spezifische Aktivität von 1 ng IL-6 war 10320 cpm.

2,5 x $10^6$ Zellen der Linie U226/Napf wurden mit verschiedenen Konzentrationen von $I^{125}$-IL-6 und dem 500-fachen Überschuß von nicht markiertem IL-6 für 90 Minuten bei 4° C in einem totalen Volumen von 1 ml PBS 1 % inkubiert. Anschließend wurde dreimal gewaschen und dann vermessen.

| IL-6 µl | gebundenes IL-6 cpm | gebundenes IL-6 ng | IL-6 total ng | freies IL-6 ng | $\dfrac{\text{geb. Il-6}}{\text{freies IL-6}}$ |
|---|---|---|---|---|---|
| 0,1 | 1900 | 0,01957 | 0,33 | 0,3104 | 0,063 |
| 0,2 | 3000 | 0,03914 | 0,66 | 0,6208 | 0,063 |
| 0,4 | 7500 | 0,07725 | 1,32 | 1,2427 | 0,063 |
| 1 | 16000 | 0,1640 | 3,33 | 3,166 | 0,052 |
| 2 | 29000 | 0,2987 | 6,66 | 6,3613 | 0,047 |
| 4 | 48000 | 0,4944 | 13,2 | 12,705 | 0,039 |
| 10 | 84000 | 0,8652 | 33 | 32,134 | 0,027 |
| 20 | 114000 | 1,1742 | 66 | 64,825 | 0,018 |
| 40 | 132000 | 1,3596 | 132 | 130,640 | 0,010 |

gebundenes IL-6 max.:    1,5 ng
0,057 x $10^{-12}$ mMol

$$\frac{0,057 \times 10^{-12} \times 6 \times 10^{23}}{2,5 \times 10^6} = 13680$$

Anzahl der Rezeptoren/Zelle:

$$kD: \quad \frac{0,057 \times 10^9}{0,063} = 8,7 \times 10^{-10} \text{ M}$$

Die Scatchard-Analyse zeigt einen kD-Wert von 8,7 x $10^{-10}$ M und 13680 Rezeptoren pro Zelle bezogen auf die Zellinie U226.

Beispiel 5: Kompetitions-Untersuchung für den Rezeptor zwischen radioaktiv markiertem IL-6 und BE-4, BE-8 und BF-6

2,5 x $10^6$ Zellen U226 per Loch wurden mit 0,1 µl (0,33 ng) von radioaktiv markiertem IL-6 inkubiert und dienten als positive Kontrolle. Der cpm-count ist 3269 ± 156 und wurde als Wert für die Gesamtbindung von IL-6 festgelegt.

Bei Zugabe des 500-fachen Überschusses von nicht markiertem IL-6 zu 0,1 µl (0,33 ng) radioaktiv markiertem IL-6 wurden 253 cpm als Wert für die nicht-spezifische Bindung gefunden. Dies ergibt als Wert für die spezifische Bindung 3016 cpm = 100 %.

Bei Durchführung des selben Experiments mit radioaktiv markiertem IL-6, aber unter Zugabe von 1 µg/ml BE-4, BE-8 oder BF-6 ergaben sich folgende Werte:

BE-4: :    11 % IL-6-Bindung
BE-8 :    14 % IL-6-Bindung
BF-6 :    92 % IL-6-Bindung

Dieses Experiment zeigt deutlich, daß BE-4 und BE-8 in der Lage sind, die spezifische Bindung von IL-6 an seinen Rezeptor zu inhibieren, BF-6 dagegen nicht in der Lage ist, diese Bindung zu inhibieren.

Beispiel 6: Scatchard-Analyse der Antikörper BE-4, BE-8 und BF-6

Jeweils 40 µg gereinigter Mak wurden mit Na$^{125}$J (0,5 mCi) in 180 µl PBS inkubiert. Anschließend wurden 10 µl (0,4 mg/ml) Chloramin T zugesetzt und die Reaktion nach 1 Minute durch Zugabe von 10 µl Natriumbisulfit (0,5 µg/ml) abgestoppt. Die so markierten Mak's wurden chromatographisch (Sephadex-G-25) von freiem Jod abgetrennt.

Für die Scatchard-Analyse wurden J$^{125}$-BE-4, J$^{125}$-BE-8 und J$^{125}$-BF-6 in einem ELISA eingesetzt;
- die Platten wurden mit 1000 ng BE-4 oder BE-8 oder BF-6 pro Napf bei 4°C über Nacht inkubiert
- anschließend wurde mit PBS 5 % Albumin abgesättigt
- anschließend wurde mit 10 ng IL-6 für 2 Stunden bei 4°C inkubiert
- anschließend wurden verschiedene Konzentrationen jodierter Antikörper und bei bestimmten Konzentrationen ebenfalls ein Überschuß an nicht-markierten Antikörpern für 90 Min bei 4°C inkubiert
- 3 x waschen und messen.

Messungen mit U266-Zellinien wurden wie folgt durchgeführt:

Durchführung wie in Beispiel 4, jedoch anstelle von jodiertem IL-6 wurde jetzt eine konstante Menge von 20 ng IL-6 pro 10$^6$ Zellen für 30 Min bei 37°C inkubiert und dann verschiedene Konzentrationen von jodiertem monoklonalen Antikörper zugesetzt und für 60 Min. bei 4°C inkubiert.

## Ergebnisse:    spezifische Aktivität:

BE-4 1 ng = 4664 cpm

BE-8 1 ng = 4571 cpm

BF-6 1 ng = 4510 cpm

| Gebundener Mak | Markierte Tracer-Mak | kD | IL-6-Moleküle |
|---|---|---|---|
| BF-6 | BE-4 | $0,8 \times 10^{-9}$ M | 15180 |
| BE-4 | BF-6 | $2,7 \times 10^{-9}$ M | 15168 |
| BE-4 | BE-8 | $0,1 \times 10^{-9}$ M | 14802 |

| | UL266 IL-6 + Antikörper | U266 Anzahl der Rezeptoren |
|---|---|---|
| BE-4 | $3,9 \times 10^{-9}$ M | 11733 |
| BF-6 | $4,7 \times 10^{-9}$ M | 27168 |
| BE-8 | $2,5 \times 10^{-9}$ M | 13425 |

Der ELISA-Scatchard zeigt identische Konzentrationen von IL-6, das in diesem System gebunden wurde für alle Antikörper, das heißt, daß die gefundenen kD-Werte einen vernünftigen Wert darstellen.

Die kD-Werte für an den Rezeptor gebundenes IL-6 auf der Zellinie U266 ist verschieden von der Konstante, die im ELISA gefunden wurde. Dies kann durch leichte Modifikationen der räumlichen Struktur von IL-6 nach der Bindung an den Rezeptor erklärt werden.

Die Befunde, daß BE-4 und BE-8 weniger an die Rezeptoren gebundenes IL-6 erkennen als BF-6, können dadurch erklärt werden, daß BE-4 und BE-8 die aktive Stelle des Moleküls erkennen und daß sie nur das IL-6-Dimer am Rezeptor erkennen können, BF-6 aber neben der dimeren auch die monomere Form erkennen kann.

Beispiel 7: Kompetitionsexperimente zwischen BE-4, BE-8 und BF-6

IL-6 (1 μg/Napf) wurde an die ELISA plates durch Inkubation bei 4° C über Nacht in Karbonatpuffer pH 9,5 gebunden. Anschließend wurden die Platten mit PBS 5 % Albumin abgesättigt und 4 x gewaschen. Dann wurde jeweils radioaktiv markierter BE-4, BE-8 und BF-6 in verschiedenen Experimenten zugegeben und inkubiert. Jeder radioaktiv markierte Antikörper wurde daneben auch mit 1 x, 10 x, 100 x Überschuß an nicht-markierte Antikörper inkubiert.

| | BE-4 J$^{125}$ 8535 cpm | | | BE-8 J$^{125}$ 9852 cpm | | | BF-6 J$^{125}$ 6514 cpm | | |
|---|---|---|---|---|---|---|---|---|---|
| | 1 x | 10 x | 100 x | 1 x | 10 x | 100 x | 1 x | 10 x | 100 x |
| BE-4 nicht markiert | 3597 | 1060 | 315 | 10517 | 9518 | 10468 | 6925 | 5621 | 6511 |
| BE-8 nicht markiert | 10810 | 8330 | 7077 | 4276 | 983 | 256 | 5974 | 4873 | 5035 |
| BF-6 nicht markiert | 8654 | 10448 | 8774 | 9518 | 8952 | 9328 | 3447 | 1130 | 703 |

Dieses Experiment zeigt, daß jeder Antikörper nur mit sich selbst kompetiert und daß keine Verdrängung zwischen BE-4, BE-8 und BF-6 stattfindet. Dies bedeutet, daß alle drei Antikörper verschiedene Epitope haben.

Beispiel 8: Sandwich-ELISA für die Messung von IL-6

- Sättigung mit BE-8 (1000 ng/Napf/100 μl) über Nacht 4° C
- Sättigung mit PBS 5 % Albumin für 90 Min bei Raumtemperatur
- Verschiedene Konzentrationen von IL-6 in Humanserum für 2 Std. bei 37° C
- Biotinylierter BE-4 (6,5 μg/Napf/100 μl, PBS, Tween 0,5 % für 90 Min bei Raumtemperatur
- Substrat, Messung

| ng IL-6/Napf/100 µl | O.D. |
|---|---|
| 100 | 1,807 |
| 10 | 1,566 |
| 1 | 1,356 |
| 0,5 | 0,921 |
| 0,25 | 0,573 |
| 0,125 | 0,401 |
| 0,062 | 0,311 |
| 0,031 | 0,212 |
| 0,015 | 0,190 |
| 0,008 | 0,195 |
| 0,004 | 0,170 |

Background:  O.D. ~ 0,150

Die Sensitivität des Tests liegt bei 0,3 ng/ml IL-6 im Serum.

Beispiel 9: Inhibition der IL-6-abhängigen humanen Zellinie GB durch die Antikörper BE-4 und BE-8

$2 \times 10^6$ Zellen der Linie GB pro Napf wurden für 5 Tage bei verschiedenen Konzentrationen an natürlichen IL-6 und verschiedene Konzentrationen an Antikörper BE-8 unter Zusatz von $H^3$-Thymidin inkubiert.

cpm

| IL-6 U/ml | 0,15 µg/ml | 0,08 µg/ml | 0,04 1g/ml | 0 µg/ml BE-8 |
|---|---|---|---|---|
| 40 | 765 | 10213 | 56432 | 78421 |
| 20 | 119 | 110 | 7237 | 77610 |
| 4 | 222 | 280 | 594 | 73197 |
| 2 | 212 | 206 | 256 | 51040 |
| 1 | 196 | 224 | 230 | 38859 |

1 U IL-6:    Die Menge an IL-6, die nötig ist, um 50 % maximale

Proliferation zu induzieren.

Identische Ergebnisse wurden für den Antikörper BE-4 erhalten. Diese Ergebnisse zeigen eine signifikante Hemmung von natürlichem IL-6 (aus Human-Lymphozyten) durch BE-8 und BE-4 an der IL-6-abhängigen humanen Zellinie GB.

## III. Erste klinische Ergebnisse mit BE-4

Drei Patienten mit einer Diagnose Multiples Myelom im Endstadium mit steigenden Zahlen von Plasmazytom-Zellen - von 50.000 auf 100.000 Plasmazytom-Zellen pro ml innerhalb von 2 Tagen - wurden mit einer Zusammensetzung aus gereinigtem BE-4 behandelt.

Die Dosis war 10 mg Pro Tag über insgesamt 4 Tage (Gesamtdosis: 40 mg). Dabei wurde die Antikörper-enthaltende Zusammensetzung in einer Konzentration von 1 mg/ml in Kochsalzlösung/1 % Humanalbumin über 30 Minuten infundiert. Es wurden keinerlei Nebenwirkungen festgestellt.

Klinische Befunde: Die Fieber-Werte sanken rasch auf normale Körpertemperatur. Das schwere Krankheitsgefühl bei allen Patienten verschwand bereits nach der ersten Applikation.

Hematologische Befunde: Die Tumormasse (Plasmazytom-Zellen) reduzierte sich sofort nach der ersten Infusion von 100.000 Zellen pro ml auf 40.000 Zellen pro ml und blieb während der gesamten Behandlungsdauer stabil.

Die Zahl der Zellen in der S-Phase (Prä-Teilungs-Phase) verminderte sich unter der Behandlung von 30 % auf 10 %.

Diese ersten klinischen Ergebnisse wurden an Patienten die sich in der Endphase der Erkrankung Multiples Myelom befanden.

Es kann deshalb erwartet werden, daß bei einer Behandlung in einem früheren Stadium der Erkrankung die Tumormasse so stark reduziert werden kann, daß andere Behandlungsmöglichkeiten (wie z. B. Knochenmarktransplantation) ermöglicht werden.

Da bis heute kein aussichtsreiches Behandlungsschema für diese Patienten verfügbar ist, wird die Behandlung mit einer Zusammensetzung aus monoklonalen Antikörpern gegen IL-6 einen neuen Ansatz ergeben, diese lethale Erkrankung zu kurieren.

Eine Behandlung mit Zusammensetzungen, hergestellt aus den Antikörpern BE-4 und BE-8 in Kombination, die verschiedene Epitope erkennen und beide die Aktivität von IL-6 hemmen, wird eine weitere Verbesserung ergeben.

Alternativ können die Antikörper BE-4, BE-8 und BF-6 an Toxine gekoppelt werden, die es dann ermöglichen, neben der Blockierung der IL-6-Aktivität die Plasmazytome durch die Fixation von IL-6 an den Rezeptor auf den Zellen abzutöten.

## Ansprüche

1. Hybridoma-Zellinien, erhältlich durch Fusion von immunisierten Milzzellen der Maus mit Maus-Myelomzellen, dadurch gekennzeichnet, daß die Milzzellen vor der Fusion gegen Interleukin-6 immunisiert worden sind und sich aus den fusionierten Zellen drei unterschiedliche Zellinien, nämlich die Hybridoma-Zellinie mit der C.N.C.M.-Hinterlegungsnummer I/913 (BE-8), die Hybridoma-Zellinie mit der C.N.C.M.-Hinterlegungsnummer I/911 (BE-4) sowie die Hybridoma-Zellinie mit der C.N.C.M.-Hinterlegungsnummer I/912 (BF-6) identifizieren und isolieren lassen, welche monoklonale Antikörper produzieren, die humanes Interleukin-6 erkennen und die in Abhängigkeit von den unterschiedlichen Zellinien jeweils an ein anderes Epitop auf dem Interleukin-6-Molekül binden.

2. Hybridoma-Zellinien nach Anspruch 1, dadurch gekennzeichnet, daß sie durch Fusion mit X63 Ag8653 Maus-Myelomzellen erhältlich sind.

3. Monoklonale Antikörper, erhältlich aus den Zellinien gemäß den Ansprüchen 1 und 2 mit einer spezifischen Bindungswirkung mit humanem Interleukin-6.

4. Monoklonale Antikörper nach Anspruch 3, dadurch gekennzeichnet, daß die von den Zellinien BE-4 und BE-8 produzierten Antikörper an den Interleukin-6-Rezeptor auf Human- und Mauszellen binden.

5. Monoklonale Antikörper nach Anspruch 3 oder 4, dadurch gekennzeichnet, daß sie Chimären bilden, wobei der konstante Teil aus humanem Ig und der variable, insbesondere der hypervariable Teil aus Maus-Ig bestehen.

6. Monoklonale Antikörper nach einem der Ansprüche 3-5, dadurch gekennzeichnet, daß sie an Toxine und/oder Chemotherapeutika gekoppelt sind.

7. Verwendung der monoklonalen Antikörper nach einem der Ansprüche 3-6 bei der Herstellung einer Komposition zur Therapie, Prophylaxe und Diagnose von Interleukin-6-abhängigen Erkrankungen, insbesondere Tumorerkrankungen, Autoimmunerkrankungen, Infektionen jeglicher Art und Störungen der Akut-Phase-Reaktionen.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,Y | EP-A-0 312 996 (T. KISHIMOTO)<br>* Ansprüche *<br>— — — | 1-4,6-7,5 | C 12 N<br>5/20<br>C 12 P 21/08<br>A 61 K 39/395<br>G 01 N 33/577<br>G 01 N 33/68 |
| X | EP-A-0 326 120 (YEDA RESEARCH & DEVELOPMENT CO., LTD.)<br>* Ansprüche *<br>— — — | 1-7 | |
| Y | JOURNAL OF IMMUNOLOGY. vol. 138, no. 12, 15 Juni 1987, BALTIMORE US Seiten 4534 - 4538; D. SHAW et al.: "Characterization of a mouse/human chimeric monoclonal antibody (17-1A) toa colon cancer tumor-associated antigen."<br>* Zusammenfassung *<br>— — — | 5 | |
| A | JOURNAL OF IMMUNOLOGY. vol. 143, no. 9, 01 November 1989, BALTIMORE US Seiten 2900 - 2906; Y. HIRATA et al.: "Characterization of IL-6 receptor expression by monoclonal and polyclonalantibodies."<br>* Zusammenfassung *<br>— — — — — | 4 | |

| RECHERCHIERTE SACHGEBIETE (Int. Cl.5) |
|---|
| C 07 K<br>C 12 P |

Der vorliegende Recherchenbericht wurde für alle Patentansprüche erstellt

| Recherchenort | Abschlußdatum der Recherche | Prüfer |
|---|---|---|
| Den Haag | 04 Februar 91 | NOOIJ F.J.M. |